# EUROPEAN PATENT SPECIFICATION

(11) **EP 4 719 611 B1**
(45) Date of publication and mention of the grant of the patent: **09.12.2026**
(21) Application number: 24730914.9
(22) Date of filing: 29.05.2024
(51) Int. Cl.: A61Q 5/08, A61K 8/19, A61K 8/25, A61K 8/41, A61K 8/22

(54) **BLEACHING COMPOSITION FOR KERATIN FIBERS**
BLEICHMITTELZUSAMMENSETZUNG FÜR KERATINFASERN
COMPOSITION DE BLANCHIMENT POUR FIBRES DE KÉRATINE

(30) Priority: 31.05.2023 EP 23176364
(43) Date of publication of application: 08.04.2026
(73) Proprietor: Kao Corporation, Tokyo 103-8210 (JP)
(72) Inventor: JIAN, Niu, 64297 Darmstadt (DE); NÖCKER, Bernd, 64297 Darmstadt (DE); BAUER, Peter, 64297 Darmstadt (DE); BREAKSPEAR, Steven, 64297 Darmstadt (DE)
(74) Representative: Hoffmann Eitle
(86) International application number: PCT/EP2024/064769
(87) International publication number: WO 2024/246128

(56) References cited:
- EP-A1- 2 883 571
- FR-A1- 3 060 349
- KR-A- 20210 059 226
- US-A- 3 823 231

## Description

### Field of the invention

The present invention is directed to a bleaching and/or lightening compositions for keratin fibers. Additionally, a method for bleaching and/or lightening and a kit-of-parts is disclosed.

### Background of the invention

Bleaching of keratin fibers is typically desired when customers with dark hair color intend to lighten their hair color. It is also necessary when the customer prefers a color shade that is lighter in comparison to their natural hair color.

A common disadvantage of all bleaching and lightening processes is that they confer a certain degree of damage to keratin fibers, and especially after repeated application, the keratin fibers lack their cosmetic appearance.

Despite the progress in recent years regarding bleaching compositions with high performance and low damage, there still is a need for further improvement.

The prior art has not yet found the ideal solution.

### Summary of the invention

Therefore, the first object of the present invention is a bleaching and/or lightening composition A for keratin fibers, preferably human keratin fibers, more preferably human hair, comprising:
a) tris-(hydroxymethyl)-aminomethane and/or 2-amino-2-methyl-1,3-propanediol, and/or their salt(s), and/or their mixtures,
b) one or more inorganic alkalizing agents,
c) one or more ammonium or alkali metal or alkaline earth metal salts of persulfates or peroxysulfates.

The second object of the present invention is a kit-of-parts for bleaching and/or lightening of keratin fibers, preferably human keratin fibers, more preferably human hair, comprising:
- a composition A as defined above, and
- a composition B comprising one or more oxidizing agent(s), preferably hydrogen peroxide.

The third object of the present invention is a method for bleaching and/or lightening of keratin fibers, preferably human keratin fibers, more preferably human hair, comprising the steps of:
i) mixing a composition A as defined above with a composition B as defined above to yield a ready-to-use composition having a pH in the range of 7 to 12,
ii) applying the ready-to-use composition onto keratin fibers and leaving it,
iii) rinsing-off the ready-to-use composition from keratin fibers.

The use of composition A as defined above reduces the damage during bleaching and/or lightening on keratin fibers, preferably human keratin fibers, more preferably human hair, with the provision that composition A is mixed with a composition B as defined above directly prior to application onto keratin fibers.

### Detailed description of the invention

The inventors of the present invention unexpectedly found that the presence of compounds in the composition, kit, method and use according to the independent claims resulted in less damage to the keratin fibers while maintaining the same bleaching performance or even increasing the bleaching performance. In addition, the bleached keratin fibers were found to have an improved cosmetic appearance with, for example, an improved touch and a softer feel.

### Bleaching and lightening composition

The present invention is directed to a bleaching and/or lightening composition A for keratin fibers, preferably human keratin fibers, more preferably human hair, comprising:
a) tris-(hydroxymethyl)-aminomethane and/or 2-amino-2-methyl-1,3-propanediol, and/or their salt(s), and/or their mixtures,
b) one or more inorganic alkalizing agents,
c) one or more ammonium or alkali metal or alkaline earth metal salts of persulfates or peroxysulfates.

### Compound(s) according to group a)

The composition of the present invention comprises tris-(hydroxymethyl)-aminomethane and/or 2-amino-2-methyl-1,3-propanediol, and/or their salt(s), and/or their mixtures as compound(s) according to group a).

The most preferred compound according to group a) is tris-(hydroxymethyl)-aminomethane, and/or its salt(s).

It is preferred from the viewpoint of bleaching performance that the total concentration of compound(s) according to group a) is 0.5% by weight or more, preferably 0.75% by weight or more, more preferably 1% by weight or more, calculated to the total weight of composition A.

It is preferred from the viewpoint of bleaching performance and damage that the total concentration of compound(s) according to group a) is 20% by weight or less, preferably 15% by weight or less, more preferably 12.5% by weight of less, calculated to the total weight of composition A.

For attaining the above-mentioned effects, it is preferred that the total concentration of compound(s) according to group a) is in the range of 0.5% to 20% by weight, preferably in the range of 0.75% to 15% by weight, more preferably in the range of 1% to 12.5% by weight, calculated to the total weight of composition A.

### Compound(s) according to group b)

Composition A of the present invention comprises one or more inorganic alkalizing agents as compound(s) according to group b).

It is preferred from the viewpoint of bleaching performance that the one or more inorganic alkalizing agent according to group b) is/are an alkali metal or alkaline earth metal salt of one or more metasilicates, one or more silicates, one or more disilicates, one or more hydroxides, one or more phosphates, one or more carbonates, one or more bicarbonates, and/or their mixtures, preferably one or more inorganic alkalizing agent according to group b) is/are an alkali metal or alkaline earth metal salt of one or more metasilicates, one or more carbonates, one or more bicarbonates, and/or their mixtures, more preferably one or more inorganic alkalizing agent according to group b) is/are sodium metasilicate, sodium carbonate, sodium bicarbonate, and/or their mixtures.

The furthermore preferred compounds according to group b) are sodium bicarbonate and sodium metasilicate, and/or their mixtures.

In one aspect of the present invention, the most preferred compound according to group b) is sodium bicarbonate.

In another aspect of the present invention, the most preferred compound according to group b) is sodium metasilicate.

It is preferred from the viewpoint of bleaching performance that the total concentration of compound(s) according to group b) is 0.25% by weight or more, preferably 0.5% by weight or more, more preferably 1% by weight or more, furthermore preferably 3% by weight or more, calculated to the total weight of composition A.

It is preferred from the viewpoint of bleaching performance and damage that the total concentration of compound(s) according to group b) is 30% by weight or less, preferably 25% by weight or less, more preferably 20% by weight of less, furthermore preferably 18% by weight or less, calculated to the total weight of composition A.

For attaining the above-mentioned technical effects, it is preferred that the total concentration of compound(s) according to group b) in composition A is in the range of 0.25% to 30% by weight, preferably in the range of 0.5% to 25% by weight, more preferably in the range of 1% to 20% by weight, furthermore preferably in the range of 3% to 18% by weight, calculated to the total weight of composition A.

It is further preferred from the viewpoint of bleaching performance and damage that the weight ratio of compound(s) according to group a) to compound(s) according to group b) is in the range of 0.1 to 10, preferably in the range of 0.2 to 8, more preferably in the range of 0.25 to 6.

### Compound(s) according to group c)

Composition A of the present invention comprises one or more ammonium or alkali metal or alkaline earth metal salts of persulfates or peroxysulfates as compound(s) according to group c).

Suitable compounds according to group c) is/are sodium persulfate, potassium persulfate, ammonium persulfate, sodium peroxymonosulfate, potassium peroxymonosulfate, and ammonium peroxymonosulfate, and/or their mixtures.

It is preferred from the viewpoint of bleaching performance that the total concentration of compound(s) according to group c) is 1% by weight or more, preferably 2% by weight or more, more preferably 5% by weight or more, calculated to the total weight of composition A.

It is preferred from the viewpoint of bleaching performance and damage that the total concentration of compound(s) according to group c) is 80% by weight or less, preferably 70% by weight or less, more preferably 60% by weight of less, calculated to the total weight of composition A.

For attaining the above-mentioned effects, it is preferred that the total concentration of compound(s) according to group c) in composition A is in the range of 1% to 80% by weight, preferably in the range of 2% to 70% by weight, more preferably in the range of 5% to 60% by weight, calculated to the total weight of composition A.

### Additional alkalizing agents

Composition A of the present invention may comprise one or more additional alkalizing agent different from compound(s) according to groups a) and b), preferably the one or more additional alkalizing agent is ammonia and/or its salt(s), an organic alkyl and/or alkanol amines having one hydroxy group, and/or their salt(s), according to the following general structure: 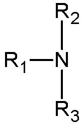 wherein R₁, R₂, and R₃ are independently selected from H, linear C₁-C₆ alkyl which may be substituted with one hydroxyl group, or branched C₃-C₁₂ alkyl or alkanol, wherein at least one of R₁, R₂, or R₃ is different from H, and/or their salts, and/or their mixtures.

Suitably, the one or more additional alkalizing agent different from compound(s) according to groups a) and b) is selected from ammonium hydroxide, monoethanolamine, diethanolamine, monoethanol methylamine, monoethanol dimethylamine, diethanolmethylamine, monoethanolethylamine, monoethanoldiethylamine, diethanolethylamine, monoethanolpropylamine, monoethanoldipropylamine, diethanolpropylamine, monoethanolbutylamine, diethanolbutylamine, trimethylamine, triethylamine, 2-amino-2-methylpropanol, and/or its/their salt(s), and/or their mixtures.

It is preferred from the viewpoint of alkalinity and bleaching power that the total concentration of one or more additional alkalizing agent different from compound(s) according to groups a) and b) is 0.25% by weight or more, preferably 0.5% by weight or more, still more preferably 1% by weight or more, calculated to the total weight of composition A.

It is preferred from the viewpoint of alkalinity and stability that the total concentration of one or more additional alkalizing agent different from compound(s) according to groups a) and b) is 30% by weight or less, preferably 25% by weight or less, still more preferably 20% by weight or less, calculated to the total weight of composition A.

For attaining the above-mentioned effects, it is preferred that the total concentration of one or more additional alkalizing agent different from compound(s) according to groups a) and b) is in the range of 0.25% to 30% by weight, preferably 0.5% to 25% by weight, more preferably 1% to 20% by weight, calculated to the total weight of composition A.

### Product forms

### Aqueous composition

In one aspect of the present invention, composition A is an aqueous composition comprising water at a total concentration of more than 10% by weight, preferably at 30% by weight or more, more preferably at 50% by weight or more, calculated to the total weight of composition A.

Preferably, from the viewpoint of bleaching efficiency, the pH of composition A is in the range of 7 to 12, more preferably in the range of 8 to 11, still more preferably in the range of 8.5 to 10.5.

The aqueous composition A may be in the form of a solution, a thickened gel, or a oil-in-water emulsion.

### Bleaching powder

It is preferred from the viewpoint of stability that the total concentration of water in composition A is 10% by weight or less, preferably 5% by weight or less, more preferably 1% by weight or less, still more preferably 0.5% by weight or less, calculated to the total weight of composition A, still more preferably composition A is an anhydrous composition.

The term 'anhydrous' does not exclude the presence of bound moisture or crystal water of any of the ingredients. 'Anhydrous' means that no water is added to composition A prior to mixing it with composition B as detailed below.

In one aspect of the present invention, composition A is a bleaching powder composition.

For this aspect, it is preferred that composition A comprises one or more pulverulent excipient, preferably one or more organic or inorganic excipient(s).

Suitable pulverulent excipients do not react with any of the compounds of composition A. Suitable ones are, for example, diatomaceous earth, kaolin, bentonite, starch especially corn, tapioca, rice, wheat and potato, nylon powder, montmorillonite, gypsum, sawdust and perlite.

For this aspect of the present invention, it is preferred from the viewpoint of powder stability that the total concentration pulverulent excipients is 10% by weight or more, more preferably 15% by weight or more, still more preferably 20% by weight or more, still more preferably 30% by weight or more, calculated to the total weight of composition A.

It is preferred from the viewpoint of powder stability that the total concentration of pulverulent excipients is 90% by weight or less, more preferably 85% by weight or less, still more preferably 80% by weight or less, calculated to the total weight of composition A.

For attaining the above-mentioned effects, it is preferred that the total concentration of pulverulent excipients is in the range of 10% to 90% by weight, more preferably 15 to 85% by weight, still more preferably in the range of 20% to 80% by weight, still more preferably in the range of 30% to 80% by weight, calculated to the total weight of composition A.

It is further preferred from the viewpoint of cosmetic safety that the bleaching powder is a dust-free bleaching powder. This is commonly achieved by adding one or more oil(s) being liquid at 25°C and under atmospheric pressure as detailed below for the bleaching paste. For the aspect of the dust-free bleaching powder, the total concentration of one or more oil(s) being liquid at 25°C and under atmospheric pressure is at 10% by weight or less, calculated to the total weight of composition A.

### Bleaching paste

In another aspect of the present invention, the composition is a bleach paste composition comprising one or more oil(s) being liquid at 25°C and under atmospheric pressure.

'Paste' within the meaning of the present invention is a dispersion of bleaching compounds in the one or more oil(s) being liquid at 25°C and under atmospheric pressure as compound(s). In most cases, the dispersion is a suspension of the bleaching compounds.

Suitable one or more oil(s) are one or more hydrocarbon oil(s), one or more ester oil(s) such as fatty acid esters consisting of linear or branched, saturated or unsaturated fatty acids with C₁₂ to C₂₂ being esterified with linear or branched primary alcohols with C₃ to C₁₂, one or more plant oil(s), silicone oil(s), and/or their mixtures.

Suitably, the one or more oil(s) being liquid at 25°C and atmospheric pressure are mineral oil, paraffinum perliquidum, paraffinum subliquidum, polydecenes, octyl palmitate, isocetyl palmitate, isopropyl palmitate, octyl stearate, oleyl oleate, myristyl myristate, almond oil, avocado oil, coconut oil, corn oil, cottonseed oil, flax seed oil, grapeseed oil, hemp oil, olive oil, palm kernel oil, peanut oil, pumpkin seed oil, rice bran oil, safflower seed oil, sesame seed oil, sunflower seed oil, soybean oil, walnut oil, and/or their mixtures.

Suitable comprises fatty compounds selected from linear or branched, saturated or unsaturated fatty alcohols with C₁₂ to C₂₂ are lauryl alcohol, tridecyl alcohol, myristyl alcohol, pentadecyl alcohol, cetyl alcohol, palmitoleyl alcohol, heptadecyl alcohol, stearyl alcohol, nonadecyl alcohol, arachidyl alcohol, behenyl alcohol, and/or their mixtures, such as cetearyl alcohol.

Suitable examples for fatty acid esters consisting of linear or branched, saturated or unsaturated fatty acids with C₁₂ to C₂₂ being esterified with linear or branched primary alcohols with C₃ to C₁₈ are octyl palmitate, isocetyl palmitate, isopropyl palmitate, octyl stearate, oleyl oleate, and myristyl myristate, as well as their mixtures.

The most preferred oil being liquid at 25°C and atmospheric pressure is isopropyl myristate.

For this aspect of the present invention, it is preferred from the viewpoint of bleaching paste stability that the total concentration of one or more oil(s) being liquid at 25°C and atmospheric pressure is more than 1% by weight or more, more preferably 2% by weight or more, still more preferably 5% by weight or more, still more preferably 10% by weight or more, calculated to the total weight of composition A.

It is preferred from the viewpoint of powder stability that the total concentration of one or more oil(s) being liquid at 25°C and atmospheric pressure is 50% by weight or less, more preferably 40% by weight or less, still more preferably 35% by weight or less, calculated to the total weight of composition A.

For attaining the above-mentioned effects, it is preferred that the total concentration of one or more oil(s) being liquid at 25°C and atmospheric pressure is in the range of 1% to 55% by weight, preferably in the range of 2% to 50% by weight, more preferably in the range of 5% to 45% by weight, still more preferably in the range of 10% to 45 % by weight, calculated to the total weight of composition A.

### Thickening polymers

It is advantageous from the viewpoint of cosmetic safety that the composition of the present invention further comprises one or more oil-soluble thickening polymer(s), preferably one or more polymer having monomer units comprising styrene, ethylene, propylene, and/or butylene.

Such polymers are offered under the trade name Kraton.

It is preferred from the viewpoint of cosmetic safety that the total concentration of one or more oil-soluble thickening polymer(s) in composition A is 0.5% by weight or more, more preferably 0.75% by weight or more, still more preferably 1% by weight or more, calculated to the total weight of composition A.

It is preferred from the viewpoint of user convenience that the total concentration of one or more oil-soluble thickening polymer(s) in composition A is 10% by weight or less, more preferably 8% by weight or less, still more preferably 6% by weight or less, calculated to the total weight of composition A.

For attaining the above-mentioned effects, it is preferred that the total concentration of one or more oil-soluble thickening polymer(s) is in the range of 0.5% to 10% by weight, preferably in the range of 0.75% to 8% by weight, more preferably in the range of 1% to 6% by weight, calculated to the total weight of composition A.

It is further preferred that composition A comprises one or more water-soluble thickening polymer(s), preferably one or more water-soluble anionic or non-ionic thickening polymer(s), still more preferably one or more polymer comprising units of acrylate, methacrylate, methacrylamide, and/or one or more xanthan gum, one or more dehydroxanthan gum, and/or one or more cellulose polymer such as methylcellulose, ethylcellulose, hydroxyethylcellulose, hydroxypropylmethylcellulose, one or more alginate polymer(s), and/or their salt(s), and/or their mixtures.

Preferably, the thickening polymers are selected from polymers resulting in an aqueous solution and/or aqueous dispersion at pH between 8 and 10 having a viscosity of at least 5,000 mPa•s measured at a polymer concentration of 1% by weight in water at 25°C, calculated to the total weight of the composition, determined by a Brookfield viscometer, such as at 10 rpm for 1 min, with an appropriate spindle at 25°C.

Suitable non-ionic thickening polymers are cellulose-based polymers. Suitable examples of cellulose-based polymers are methylcellulose, hydroxymethylcellulose, hydroxyethylcellulose, hydroxypropylcellulose, hydroxyethyl-methylcellulose, and alkylated hydroxyl celluloses such as (C2-C8)-alkylcelluloses or cetyl hydroxyethylcellulose.

Suitable anionic thickening polymers are selected from naturally-based anionic polymers and/or synthetic anionic polymers.

Suitably, the natural anionic polymer(s) may be selected from xanthan gum, dehydroxanthan gum, hydroxypropylxanthan gum, carboxymethyl cellulose and starch based polymers such as vegetable starch and/or their synthetically modified derivatives such as hydroxypropyl starch phosphate. Equally suitable are alginic acids, sodium alginates, ammonium alginates, calcium alginates, gum arabic, and guar gum.

The preferred thickening polymer for the composition of the present invention are natural anionic polymers, more preferably xanthan gum and/or dehydroxanthan gum, from the viewpoint of their biodegradability and low environmental impact.

Preferably, the total concentration of water-soluble thickening polymers of the present invention are 0.1% by weight or more, more preferably 0.25% by weight or more, more preferably 0.5% by weight or more, calculated to the total weight of the composition, from the viewpoint of providing sufficient viscosity to the composition.

Preferably, the total concentration of water-soluble thickening polymers of the present invention are 15% by weight or less, more preferably 12% by weight or more, still more preferably 10% by weight or less, calculated to the total weight of the composition, from the viewpoint of providing sufficient viscosity to the composition and cost of goods.

For attaining the above-mentioned effects, it is preferred that the total concentration of water-soluble thickening polymers in the composition of the present invention is in the range of 0.1% to 15% by weight, preferably 0.25% to 12% by weight, more preferably in the range of 0.5% to 10% by weight, calculated to the total weight of the composition.

### Optional ingredients

### Surfactants

It is further preferred from the viewpoint of mixability of composition A and wetting of keratin fibers that composition A further comprises one or more surfactant(s), more preferably selected from non-ionic, cationic, anionic, zwitterionic/amphoteric surfactant(s).

Anionic surfactants suitable are in principle known from the cleansing compositions. These are anionic surfactants of the sulfate, sulfonate, carboxylate and alkyl phosphate type, for example, the known C₁₀-C₁₈-alkyl sulfates, and in particular the respective ether sulfates, for example, C₁₂-C₁₄-alkyl ether sulfate, lauryl ether sulfate, especially with 1 to 4 ethylene oxide groups in the molecule, monoglyceride (ether) sulfates, fatty acid amide sulfates obtained by ethoxylation and subsequent sulfatation of fatty acid alkanolamides, and the alkali salts thereof, as well as the salts of long-chain mono- and dialkyl phosphates. Preferred anionic surfactants are alkyl sulphate surfactants especially lauryl sulphate and its salts.

Further suitable surfactants are nonionic surfactants. Non-limiting examples are long-chain fatty acid mono- and dialkanolamides, such as coco fatty acid mono- or diethanolamide and myristic fatty acid mono or diethanolamide, stearic acid mono or diethanolamide, alkyl polyglucosides with an alkyl group of 8 to 18 carbon atoms, and with 1 to 5 glucoside units, sorbitan esters, such as polyethylene glycol sorbitan stearic, palmitic, myristic and lauric acid esters, fatty acid polyglycol esters or polycondensates of ethyleneoxide and propyleneoxide, as they are on the market, for example, under the trade name "Pluronics^{R}", as well as fatty alcohol ethoxylates, C₁₀-C₂₂-fatty alcohol ethoxylates, known by the generic terms "Laureth", "Myristeth", "Oleth", "Ceteth", "Deceth", "Steareth" and "Ceteareth" according to the CTFA nomenclature, including addition of the number of ethylene oxide molecules, e.g., "Laureth-16": The average degree of ethoxylation thereby ranges between about 2.5 and about 100, preferably about 10 and about 30.

Suitable amphoteric/zwitterionic surfactants are in particular the various known betaines such as alkyl betaines, fatty acid amidoalkyl betaines and sulfobetaines, for example, lauryl hydroxysulfobetaine; long-chain alkyl amino acids, such as cocoaminoacetate, cocoaminopropionate and sodium cocoamphopropionate and - acetate are also suitable.

Typical cationic surfactants are cetyl trimethyl ammonium chloride, stearyl trimonium chloride, dipalmitoyl dimonium chloride, distearyl dimethyl ammonium chloride, stearamidopropyl trimonuim chloride, dioleoylethyl dimethyl ammonium methosulfate, dioleoylethyl hydroxyethylmonium methosulfate.

It is preferred from the viewpoint of composition stability, wetting of keratin fibers, and ingredient dispersability that the total concentration of surfactants is 0.1% by weight or more, preferably 0.2% by weight or more, still more preferably 0.25% by weight or more, calculated to the total weight of the composition A.

It is preferred from the viewpoint of composition stability, wetting of keratin fibers, and ingredient dispersability that the total concentration of surfactants is 35% by weight or less, preferably 30% by weight or less, still more preferably 25% by weight or less, calculated to the total weight of the composition A.

For attaining the above-mentioned effects, it is preferred that the total concentration of surfactants is in the range of 0.1% to 35% by weight, more preferably 0.2% to 30% by weight, still more preferably 0.25% to 25% by weight, calculated to the total weight of the composition A.

### Kit-of-parts

The present invention is also directed to a kit-of-parts for bleaching and/or lightening of keratin fibers, preferably human keratin fibers, more preferably human hair, comprising:
- a composition A as defined above, and
- a composition B comprising one or more oxidizing agent(s), preferably hydrogen peroxide.

The preferred oxidizing agent of composition B is hydrogen peroxide, from the viewpoint of bleaching performance.

Composition B preferably comprises hydrogen peroxide at a total concentration in the range of 0.1% to 20% by weight, preferably 0.25% to 15% by weight, and more preferably 0.5% to 12% by weight, calculated to the total weight of composition B.

In this respect, it is preferred that composition B is an aqueous composition, preferably comprising water at 50% by weight or more, more preferably at 60% by weight or more, still more preferably at 70% by weight or more, calculated to the total weight of composition B.

The pH of composition B preferably is in the range of 1.5 to 5, more preferably in the range of 2 to 4.5, adjusted by suitable acids and bases.

Compositions A and B in this aspect of the present invention are intended to be mixed directly prior to application onto keratin fibers. Upon mixture, the cosmetic form of the ready-to-use composition is a cream, i.e., an emulsion, typically in the form of an oil-in-water emulsion.

### Method of bleaching and/or lightening

The present invention is also directed to a method for bleaching and/or lightening of keratin fibers, preferably human keratin fibers, more preferably human hair, comprising the steps of:
i) mixing a composition A as defined above with a composition B as defined above to yield a ready-to-use composition having a pH in the range of 7 to 12,
ii) applying the ready-to-use composition onto keratin fibers and leaving it,
iii) rinsing-off the ready-to-use composition from keratin fibers.

It is preferred from the viewpoint of bleaching performance that the pH of the ready-to-use composition as defined in step i) is in the range of 8 to 11, more preferably in the range of 8.5 to 10.5.

It is further preferred from the viewpoint of bleaching performance economy that the leave-on time as defined in step ii) is in the range of 2 min to 45 min, more preferably in the range of 5 min to 40 min, still more preferably in the range of 10 min to 30 min.

The present disclosure is also directed to <1> a bleaching and/or lightening composition A for keratin fibers, preferably human keratin fibers, more preferably human hair, comprising:
a) tris-(hydroxymethyl)-aminomethane and/or 2-amino-2-methyl-1,3-propanediol, and/or their salt(s), and/or their mixtures,
b) one or more inorganic alkalizing agents,
c) one or more ammonium or alkali metal or alkaline earth metal salts of persulfates or peroxysulfates.

<2> The composition according to clause <1> characterized in that one or more compound(s) according to group a) is tris-(hydroxymethyl)-aminomethane, and/or its salt(s).

<3> The composition according to any of the clauses <1> to <2> characterized in that the total concentration of compound(s) according to group a) is in the range of 0.5% to 20% by weight, preferably in the range of 0.75% to 15% by weight, more preferably in the range of 1% to 12.5% by weight, calculated to the total weight of composition A.

<4> The composition according to any of the clauses <1> to <2> characterized in that the total concentration of tris-(hydroxymethyl)-aminomethane, and/or its salt(s) in composition A is in the range of 0.5% to 20% by weight, preferably in the range of 0.75% to 15% by weight, more preferably in the range of 1% to 12.5% by weight, calculated to the total weight of composition A.

<5> The composition according to any of the clauses <1> to <4> characterized in that the one or more inorganic alkalizing agent according to group b) is/are an alkali metal or alkaline earth metal salt of one or more metasilicates, one or more silicates, one or more disilicates, one or more hydroxides, one or more phosphates, one or more carbonates, one or more bicarbonates, and/or their mixtures.

<6> The composition according to any of the clauses <1> to <5> characterized in that the one or more inorganic alkalizing agent according to group b) is/are an alkali metal or alkaline earth metal salt of one or more metasilicates, one or more carbonates, one or more bicarbonates, and/or their mixtures.

<7> The composition according to any of the clauses <1> to <5> characterized in that the one or more inorganic alkalizing agent according to group b) is/are sodium metasilicate, sodium carbonate, sodium bicarbonate, and/or their mixtures.

<8> The composition according to any of the clauses <1> to <7> characterized in that the total concentration of compound(s) according to group b) in composition A is in the range of 0.25% to 30% by weight, preferably in the range of 0.5% to 25% by weight, more preferably in the range of 1% to 20% by weight, furthermore preferably in the range of 3% to 18% by weight, calculated to the total weight of composition A.

<9> The composition according to any of the clauses <1> to <8> characterized in that the one or more compound(s) according to group c) is/are sodium persulfate, potassium persulfate, ammonium persulfate, sodium peroxymonosulfate, potassium peroxymonosulfate, ammonium peroxymonosulfate, and/or their mixtures.

<10> The composition according to any of the clauses <1> to <9> characterized in that the total concentration of compound(s) according to group c) in composition A is in the range of 1% to 80% by weight, preferably in the range of 2% to 70% by weight, more preferably in the range of 5% to 60% by weight, calculated to the total weight of composition A.

<11> The composition according to any of the clauses <1> to <10> characterized in that the weight ratio of compound(s) according to group a) to compound(s) according to group b) is in the range of 0.1 to 10, preferably in the range of 0.2 to 8, more preferably in the range of 0.25 to 6.

<12> The composition according to any of clauses <1> to <11> characterized in that the total concentration of water in composition A is 10% by weight or less, preferably 5% by weight or less, more preferably 1% by weight or less, still more preferably 0.5% by weight or less, calculated to the total weight of composition A, still more preferably composition A is an anhydrous composition.

<13> The composition according to any of the clauses <1> to <12> characterized in that composition A is a bleach powder composition, and preferably comprises one or more pulverulent excipient, more preferably one or more organic or inorganic excipient(s), still more preferably one or more diatomaceous earth, kaolin, bentonite, starch especially corn, tapioca, rice, wheat and potato, nylon powder, montmorillonite, gypsum, sawdust, and perlite, and/or their mixtures.

<14> The composition according to any of the clauses <1> to <13> characterized in that composition A comprises one or more oil(s) being liquid at 25°C and atmospheric pressure.

<15> The composition according to any clause <14> characterized in that the one or more oil(s) being liquid at 25°C and atmospheric pressure is/are one or more hydrocarbon oil(s), one or more ester oil(s) such as fatty acid esters consisting of linear or branched, saturated or unsaturated fatty acids with C₁₂ to C₂₂ being esterified with linear or branched primary alcohols with C₃ to C₁₂, one or more plant oil(s), one or more silicone oil(s), and/or their mixtures.

<16> The composition according to any of the clauses <14> to <15> characterized in that the one or more oil(s) being liquid at 25°C and atmospheric pressure is/are mineral oil, paraffinum perliquidum, paraffinum subliquidum, polydecenes, octyl palmitate, isocetyl palmitate, isopropyl palmitate, octyl stearate, oleyl oleate, myristyl myristate, almond oil, avocado oil, coconut oil, corn oil, cottonseed oil, flax seed oil, grapeseed oil, hemp oil, olive oil, palm kernel oil, peanut oil, pumpkin seed oil, rice bran oil, safflower seed oil, sesame seed oil, sunflower seed oil, soybean oil, walnut oil, and/or their mixtures.

<17> The composition according to any of the clauses <14> to <16> characterized in that the total concentration of one or more oil(s) being liquid at 25°C and atmospheric pressure is in the range of 1% to 55% by weight, preferably in the range of 2% to 50% by weight, more preferably in the range of 5% to 45% by weight, still more preferably in the range of 10% to 45 % by weight, calculated to the total weight of composition A.

<18> The composition according to any of the clauses <1> to <12> and <14> to <17> characterized in that composition A is a is a bleach paste composition.

<19> The composition according to any of the clauses <1> to <11> characterized in that composition A is an aqueous composition comprising water at a total concentration of more than 10% by weight, preferably at 30% by weight or more, more preferably at 50% by weight or more, calculated to the total weight of composition A.

<20> The composition according to any of the clauses <1> to <11> and/or <19> characterized in that the pH of composition A is in the range of 7 to 12, more preferably in the range of 8 to 11, still more preferably in the range of 8.5 to 10.5.

<21> The composition according to any of the clauses <1> to <20> characterized in that composition A comprises one or more water-soluble thickening polymer(s), preferably one or more water-soluble anionic or non-ionic thickening polymer(s), still more preferably one or more polymer comprising units of acrylate, methacrylate, methacrylamide, and/or one or more xanthan gum, one or more dehydroxanthan gum, and/or one or more cellulose polymer such as methylcellulose, ethylcellulose, hydroxyethylcellulose, hydroxypropylmethylcellulose, one or more alginate polymer(s), and/or their salt(s), and/or their mixtures.

<22> The composition according to clause <21> characterized in that the total concentration of one or more water-soluble thickening polymer(s) in composition A is in the range of 0.1% to 15% by weight, preferably 0.25% to 12% by weight, more preferably in the range of 0.5% to 10% by weight, calculated to the total weight of composition A.

<23> The composition according to any of the clauses <1> to <22> characterized in that composition A further comprises one or more surfactant(s), preferably selected from non-ionic, cationic, anionic, zwitterionic/amphoteric surfactant(s).

<24> The composition according to clause <23> characterized in that the total concentration of surfactants is in the range of 0.1% to 35% by weight, more preferably 0.2% to 30% by weight, still more preferably 0.25% to 25% by weight, calculated to the total weight of the composition A.

<25> The composition according to any of the clauses <1> to <24> characterized in that composition A comprises one or more additional alkalizing agent different from compound(s) according to groups a) and b), preferably the one or more additional alkalizing agent is ammonia and/or its salt(s), an organic alkyl and/or alkanol amines having one hydroxy group, and/or their salt(s) according to the following general structure: 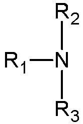 wherein R1, R2, and R3 are independently selected from H, linear C1-C6 alkyl which may be substituted with one hydroxyl group, or branched C3-C12 alkyl or alkanol, wherein at least one of R1, R2, or R3 is different from H, and/or their salts, and/or their mixtures.

<26> The composition according to clause <25> characterized in that the one or more additional alkalizing agent different from compound(s) according to groups a) and b) is/are selected from ammonium hydroxide, monoethanolamine, diethanolamine, monoethanol methylamine, monoethanol dimethylamine, diethanolmethylamine, monoethanolethylamine, monoethanoldiethylamine, diethanolethylamine, monoethanolpropylamine, monoethanoldipropylamine, diethanolpropylamine, monoethanolbutylamine, diethanolbutylamine, trimethylamine, triethylamine, 2-amino-2-methylpropanol, and/or its/their salt(s), and/or their mixtures.

<27> The composition according to any of the clauses <25> to <26> characterized in that the total concentration of one or more additional alkalizing agent different from compound(s) according to groups a) and b) is in the range of 0.25% to 30% by weight, preferably 0.5% to 25% by weight, more preferably 1% to 20% by weight, calculated to the total weight of composition A.

<28> The composition according to any of the clauses <1> to <27> characterized in that composition A comprises one or more oil-soluble thickening polymer(s).

<29> The composition according to clause <28> characterized in that one or more oil-soluble thickening polymer(s) have monomer units comprising styrene, ethylene, propylene, and/or butylene.

<30> The composition according to any of the clauses <28> to <29> characterized in that the total concentration of one or more oil-soluble thickening polymer(s) is in the range of 0.5% to 10% by weight, preferably in the range of 0.75% to 8% by weight, more preferably in the range of 1% to 6% by weight, calculated to the total weight of composition A.

The present disclosure is also directed to <31> a kit-of-parts for bleaching and/or lightening of keratin fibers, preferably human keratin fibers, more preferably human hair, comprising:
- a composition A as defined in any of the clauses <1> to <30>, and
- a composition B comprising one or more oxidizing agent(s), preferably hydrogen peroxide.

<32> The kit according to clause <31> characterized in that the total concentration of oxidizing agents, preferably the total concentration of hydrogen peroxide, in Composition B is in the range of 0.1% to 20% by weight, preferably 0.25% to 15% by weight, and more preferably 0.5% to 12% by weight, calculated to the total weight of composition B.

<33> The kit according to any of the clauses <31> to <32> characterized in that the pH of composition B is in the range of 1.5 to 5, preferably in the range of 2 to 4.5, adjusted by suitable acids and bases.

<34> The kit according to any of the clauses <31> to <33> characterized in that compositions A and B are kept separate and are mixed directly prior to application onto keratin fibers.

The present disclosure is also directed to <35> a method for bleaching and/or lightening of keratin fibers, preferably human keratin fibers, more preferably human hair, comprising the steps of:
i) mixing a composition A as defined in any of the clauses <1> to <34> with a composition B as defined in any of the clauses <31> to <34> to yield a ready-to-use composition having a pH in the range of 7 to 12,
ii) applying the ready-to-use composition onto keratin fibers and leaving it,
iii) rinsing-off the ready-to-use composition from keratin fibers.

<36> The method according to clause <35> characterized in that the pH of the ready-to-use composition as defined in step i) is in the range of 8 to 11, preferably in the range of 8.5 to 10.5.

<37> The method according to any of the clauses <35> to <36> characterized in that the leave-on time as defined in step ii) is in the range of 2 min to 45 min, preferably in the range of 5 min to 40 min, more preferably in the range of 10 min to 30 min.

The following examples are to illustrate the present invention, but not to limit it.

### EXAMPLES

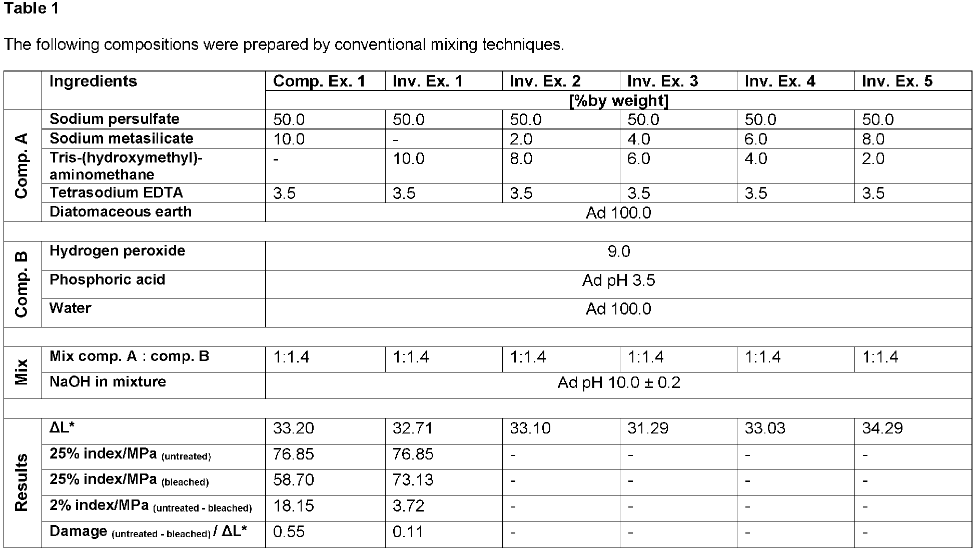

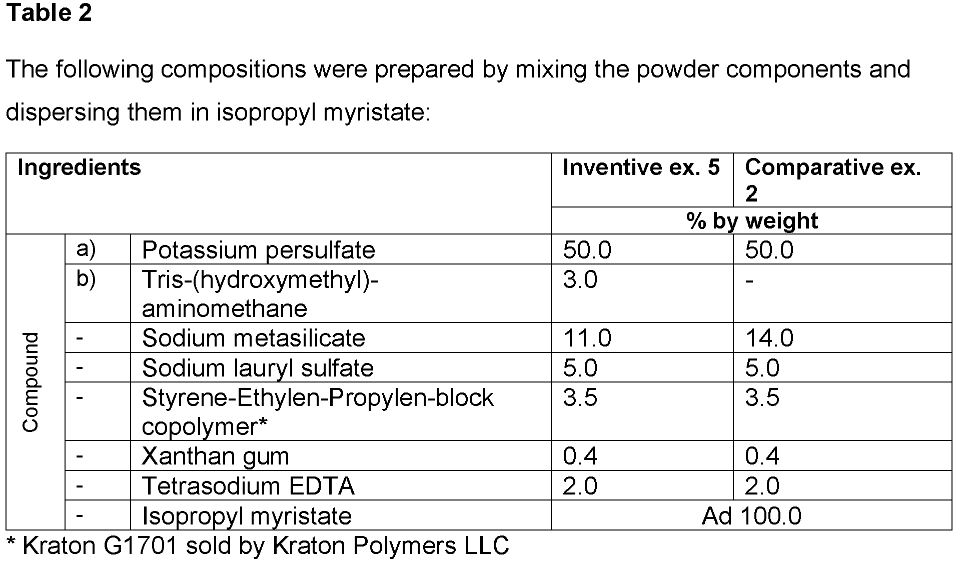

The bleach oil composition from above was mixed with the following oxidizing composition in a weight ratio of 1:1.4 directly prior to application onto hair:

| | **% by weight** |
|---|---|
| Hydrogen peroxide | 9.0 |
| Phosphoric acid | ad pH 3.5 |
| Water | ad 100.0 |

The resulting ready-to-use mixture had a pH around 9.5.

The following bleaching and coloring results were obtained:

| **Hair type** | | **Inventive ex. 5** | **Comparative ex. 2** |
|---|---|---|---|
| **L6 hair** | **ΔL*** | 43.49 | 34.56 |
| | **2% index/MPa _{(untreated)}** | 49.40 | 49.40 |
| | **2% index/MPa _{(bleached)}** | 26.29 | 24.84 |
| | **2% index/MPa _{(untreated -} _{bleached)}** | 23.11 | 24.56 |
| | **Damage _{(untreated - bleached)} / ΔL*** | 0.53 | 0.71 |
| | **Feel** | Smooth, easy combing | Rough, difficult to comb through |

### Methods

### Hair bleaching

Human level 6 hair (21 cm long, 2 g per bundle) were purchased from International Hair Importers, Glendale, NY, USA. The hair was shampooed prior to treatment.

2 g of each of the compositions from above were applied onto a hair streak and left for 30 min at 50°C. Then the hair streaks were rinsed off with water, shampooed, and blow-dried.

### Lightening measurements

L values were measured before and after bleaching with a Datacolor 45G instrument. ΔL was calculated by subtracting the L value before bleaching from the L value after bleaching.

### Damage measurements

Damage was assessed, for twenty-five individual European hair fibres per treatment, via tensile test using an MTT680 instrument (Dia-Stron Limited, Andover, UK), in the wet condition. The initial sample length used was thirty millimetres and the strain rate was 100 percent per minute. The damage value is expressed as the stress (in MPa) at two percent or twenty five percent strain, where the higher number damage/lift (Damage _{(untreated} - _{bleached)} / ΔL*) indicates higher damage.

The following examples are within the scope of the present invention.

### Inv. Ex. 6

### Composition A

| | **% by weight** |
|---|---|
| Sodium persulfate | 20.0 |
| Ammonium persulfate | 15.0 |
| Tris-(hydroxymethyl)-aminomethane | 5.0 |
| Sodium metasilicate | 5.0 |
| Sodium lauryl sulfate | 3.0 |
| Tetrasodium EDTA | 1.5 |
| Diatomaceous earth | ad 100.0 |

The bleach powder composition is mixed with the oxidative composition B of table 1 in a weight ratio of 1:1.4 (composition A : composition B).

### Inv. Ex. 7

### Composition A

| | **% by weight** |
|---|---|
| Sodium persulfate | 20.0 |
| Ammonium persulfate | 15.0 |
| Tris-(hydroxymethyl)-aminomethane | 5.0 |
| Sodium metasilicate | 5.0 |
| Sodium lauryl sulfate | 3.0 |
| Tetrasodium EDTA | 1.5 |
| Styrene-Ethylen-Propylen-block copolymer | 1.0 |
| Xanthan gum | 0.5 |
| Isopropyl myristate | ad 100.0 |

The bleach powder composition is mixed with the oxidative composition B of table 1 in a weight ratio of 1:1.4 (composition A : composition B).

### Inv. Ex. 8

### Composition A

| | **% by weight** |
|---|---|
| Tris-(hydroxymethyl)-aminomethane | 10.0 |
| Sodium bicarbonate | 2.0 |
| Cetearyl alcohol | 6.0 |
| Mineral oil | 2.0 |
| Sodium lauryl sulfate | 3.0 |
| Tetrasodium EDTA | 0.25 |
| Xanthan gum | 0.5 |
| Water | ad 100.0 |

The bleaching composition is mixed with the oxidative composition B of table 1 in a weight ratio of 1:1.4 (composition A : composition B).

## Claims

1. A bleaching and/or lightening composition A for keratin fibers, preferably human keratin fibers, more preferably human hair, comprising:
a) tris-(hydroxymethyl)-aminomethane and/or 2-amino-2-methyl-1,3-propanediol, and/or their salt(s), and/or their mixtures,
b) one or more inorganic alkalizing agents,
c) one or more ammonium or alkali metal or alkaline earth metal salts of persulfates or peroxysulfates.

2. The composition according to claim 1 **characterized in that** the one or more compound(s) according to group a) is tris-(hydroxymethyl)-aminomethane, and/or its salt(s).

3. The composition according to any of the claims 1 and/or 2 **characterized in that** the total concentration of compound(s) according to group a) is in the range of 0.5% to 20% by weight, preferably in the range of 0.75% to 15% by weight, more preferably in the range of 1% to 12.5% by weight, calculated to the total weight of composition A.

4. The composition according to any of the preceding claims **characterized in that** the one or more inorganic alkalizing agent according to group b) is/are an alkali metal or alkaline earth metal salt of one or more metasilicates, one or more silicates, one or more disilicates, one or more hydroxides, one or more phosphates, one or more carbonates, one or more bicarbonates, and/or their mixtures, preferably the one or more inorganic alkalizing agent according to group b) is/are an alkali metal or alkaline earth metal salt of one or more metasilicates, one or more carbonates, one or more bicarbonates, and/or their mixtures, more preferably one or more inorganic alkalizing agent according to group b) is/are sodium metasilicate, sodium carbonate, sodium bicarbonate, and/or their mixtures.

5. The composition according to any of the preceding claims **characterized in that** the total concentration of compound(s) according to group b) in composition A is in the range of 0.25% to 30% by weight, preferably in the range of 0.5% to 25% by weight, more preferably in the range of 1% to 20% by weight, furthermore preferably in the range of 3% to 18% by weight, calculated to the total weight of composition A.

6. The composition according to any of the preceding claims **characterized in that** the one or more compound(s) according to group c) is/are sodium persulfate, potassium persulfate, ammonium persulfate, sodium peroxymonosulfate, potassium peroxymonosulfate, and ammonium peroxymonosulfate, and/or their mixtures, and preferably the total concentration of compound(s) according to group c) in the composition A is in the range of 1% to 80% by weight, preferably in the range of 2% to 70% by weight, more preferably in the range of 5% to 60% by weight, calculated to the total weight of composition A.

7. The composition according to any of the preceding claims **characterized in that** the weight ratio of compound(s) according to group a) to compound(s) according to group b) is in the range of 0.1 to 10, preferably in the range of 0.2 to 8, more preferably in the range of 0.25 to 6.

8. The composition according to any of the preceding claims **characterized in that** the total concentration of water in composition A is 10% by weight or less, preferably 5% by weight or less, more preferably 1% by weight or less, still more preferably 0.5% by weight or less, calculated to the total weight of composition A, still more preferably composition A is an anhydrous composition.

9. The composition according to any of the preceding claims **characterized in that** composition A is a bleach powder composition, and preferably comprises one or more pulverulent excipient, more preferably one or more organic or inorganic excipient(s), still more preferably one or more diatomaceous earth, kaolin, bentonite, starch especially corn, tapioca, rice, wheat and potato, nylon powder, montmorillonite, gypsum, sawdust, and perlite, and/or their mixtures.

10. The composition according to any of the preceding claims **characterized in that** composition A comprises one or more oil(s) being liquid at 25°C and atmospheric pressure, preferably the one or more oil(s) is one or more hydrocarbon oil(s), one or more ester oil(s) such as fatty acid esters consisting of linear or branched, saturated or unsaturated fatty acids with C₁₂ to C₂₂ being esterified with linear or branched primary alcohols with C₃ to C₁₂, one or more plant oil(s), one or more silicone oil(s), and/or their mixtures, preferably the one or more oil(s) being liquid at 25°C and atmospheric pressure are mineral oil, paraffinum perliquidum, paraffinum subliquidum, polydecenes, octyl palmitate, isocetyl palmitate, isopropyl palmitate, octyl stearate, oleyl oleate, myristyl myristate, almond oil, avocado oil, coconut oil, corn oil, cottonseed oil, flax seed oil, grapeseed oil, hemp oil, olive oil, palm kernel oil, peanut oil, pumpkin seed oil, rice bran oil, safflower seed oil, sesame seed oil, sunflower seed oil, soybean oil, walnut oil, and/or their mixtures.

11. The composition according to any of the claims 1 to 8 and/or 10 **characterized in that** composition A is a bleach paste composition.

12. The composition according to any of the claims 1 to 7 and/or 10 **characterized in that** composition A is an aqueous composition comprising water at a total concentration of more than 10% by weight, preferably at 30% by weight or more, more preferably at 50% by weight or more, calculated to the total weight of composition A, and preferably the pH of composition A is in the range of 7 to 12, more preferably in the range of 8 to 11, still more preferably in the range of 8.5 to 10.5.

13. The composition according to any of the preceding claims **characterized in that** composition A comprises one or more water-soluble thickening polymer(s), preferably one or more water-soluble anionic or non-ionic thickening polymer(s), still more preferably one or more polymer comprising units of acrylate, methacrylate, methacrylamide, and/or one or more xanthan gum, one or more dehydroxanthan gum, and/or one or more cellulose polymer such as methylcellulose, ethylcellulose, hydroxyethylcellulose, hydroxypropylmethylcellulose, one or more alginate polymer(s), and/or their salt(s), and/or their mixtures.

14. A kit-of-parts for bleaching and/or lightening of keratin fibers, preferably human keratin fibers, more preferably human hair, comprising:
- a composition A as defined in any of the claims 1 to 13, and
- a composition B comprising one or more oxidizing agent(s), preferably hydrogen peroxide.

15. A method for bleaching and/or lightening of keratin fibers, preferably human keratin fibers, more preferably human hair, comprising the steps of:
i) mixing a composition A as defined in any of the claims 1 to 13 with a composition B as defined in claim 14 to yield a ready-to-use composition having a pH in the range of 7 to 12,
ii) applying the ready-to-use composition onto keratin fibers and leaving it,
iii) rinsing-off the ready-to-use composition from keratin fibers.

## Patentansprüche

1. Bleich- und/oder Aufhellungszusammensetzung A für Keratinfasern, bevorzugt menschliche Keratinfasern, bevorzugter menschliches Haar, umfassend:
a) Tris-(hydroxymethyl)-aminomethan und/oder 2-Amino-2-methyl-1,3-propandiol und/oder deren Salz(e) und/oder deren Mischungen,
b) ein oder mehrere anorganische Alkalisierungsmittel,
c) ein oder mehrere Ammonium-, Alkalimetall- oder Erdalkalimetallsalze der Persulfate oder Peroxysulfate.

2. Zusammensetzung gemäß Anspruch 1, **dadurch gekennzeichnet, dass** die eine oder mehreren Verbindung(en) gemäß Gruppe a) Tris-(hydroxymethyl)-aminomethan und/oder deren Salz(e) ist/sind.

3. Zusammensetzung gemäß einem der Ansprüche 1 und/oder 2, **dadurch gekennzeichnet, dass** die Gesamtkonzentration der Verbindung(en) gemäß Gruppe a) im Bereich von 0,5 bis 20 Gew.-%, bevorzugt im Bereich von 0,75 bis 15 Gew.-%, bevorzugter im Bereich von 1 bis 12,5 Gew.-%, bezogen auf das Gesamtgewicht der Zusammensetzung A, liegt.

4. Zusammensetzung gemäß einem der vorhergehenden Ansprüche, **dadurch gekennzeichnet, dass** das eine oder die mehreren anorganischen Alkalisierungsmittel gemäß Gruppe b) ein Alkalimetall- oder Erdalkalimetallsalz eines oder mehrerer Metasilikate, eines oder mehrerer Silikate, eines oder mehrerer Disilikate, eines oder mehrerer Hydroxide, eines oder mehrerer Phosphate, eines oder mehrerer Carbonate, eines oder mehrerer Bicarbonate, und/oder deren Mischungen ist/sind; das eine oder die mehreren anorganischen Alkalisierungsmittel gemäß Gruppe b) bevorzugt ein Alkalimetall- oder Erdalkalimetallsalz eines oder mehrerer Metasilikate, eines oder mehrerer Carbonate, eines oder mehrerer Bicarbonate und/oder deren Mischungen ist/sind; das eine oder die mehreren anorganischen Alkalisierungsmittel gemäß Gruppe b) bevorzugter Natriummetasilikat, Natriumcarbonat, Natriumbicarbonat und/oder deren Mischungen ist/sind.

5. Zusammensetzung gemäß einem der vorhergehenden Ansprüche, **dadurch gekennzeichnet, dass** die Gesamtkonzentration der Verbindung(en) gemäß Gruppe b) in der Zusammensetzung A im Bereich von 0,25 bis 30 Gew.-%, bevorzugt im Bereich von 0,5 bis 25 Gew.-%, bevorzugter im Bereich von 1 bis 20 Gew.-%, noch bevorzugter im Bereich von 3 bis 18 Gew.-%, bezogen auf das Gesamtgewicht der Zusammensetzung A, liegt.

6. Zusammensetzung gemäß einem der vorhergehenden Ansprüche, **dadurch gekennzeichnet, dass** die eine oder mehreren Verbindung(en) gemäß Gruppe c) Natriumpersulfat, Kaliumpersulfat, Ammoniumpersulfat, Natriumperoxymonosulfat, Kaliumperoxymonosulfat, und Ammoniumperoxymonosulfat und/oder deren Mischungen ist/sind, und bevorzugt die Gesamtkonzentration der Verbindung(en) gemäß Gruppe c) in der Zusammensetzung A im Bereich von 1 bis 80 Gew.-%, bevorzugt im Bereich von 2 bis 70 Gew.-%, bevorzugter im Bereich von 5 bis 60 Gew.-%, bezogen auf das Gesamtgewicht der Zusammensetzung A, liegt.

7. Zusammensetzung gemäß einem der vorhergehenden Ansprüche, **dadurch gekennzeichnet, dass** das Gewichtsverhältnis der Verbindung(en) gemäß Gruppe a) zu der/den Verbindung(en) gemäß Gruppe b) im Bereich von 0,1 bis 10, bevorzugt im Bereich von 0,2 bis 8, bevorzugter im Bereich von 0,25 bis 6 liegt.

8. Zusammensetzung gemäß einem der vorhergehenden Ansprüche, **dadurch gekennzeichnet, dass** die Gesamtkonzentration an Wasser in der Zusammensetzung A 10 Gew.-% oder weniger, bevorzugt 5 Gew.-% oder weniger, bevorzugter 1 Gew.-% oder weniger, noch bevorzugter 0,5 Gew.-% oder weniger, bezogen auf das Gesamtgewicht der Zusammensetzung A, beträgt, noch bevorzugter ist die Zusammensetzung A eine wasserfreie Zusammensetzung.

9. Zusammensetzung gemäß einem der vorhergehenden Ansprüche, **dadurch gekennzeichnet, dass** die Zusammensetzung A eine Bleichpulverzusammensetzung ist und bevorzugt einen oder mehrere pulverförmige Hilfsstoffe, bevorzugter einen oder mehrere organische oder anorganische Hilfsstoffe, noch bevorzugter einen oder mehrere aus Kieselgur, Kaolin, Bentonit, Stärke, insbesondere Mais-, Tapioka- Reis-, Weizen- oder Kartoffelstärke, Nylonpulver, Montmorillonit, Gips, Sägemehl und Perlit und/oder deren Mischungen umfasst.

10. Zusammensetzung gemäß einem der vorhergehenden Ansprüche, **dadurch gekennzeichnet, dass** die Zusammensetzung A ein oder mehrere Öl(e) umfasst, das/die bei 25 °C und atmosphärischem Druck flüssig ist/sind, wobei das eine oder die mehrere Öl(e) bevorzugt ein oder mehrere Kohlenwasserstofföl(e), ein oder mehrere Esteröl(e), wie beispielsweise Fettsäureester, bestehend aus linearen oder verzweigten, gesättigten oder ungesättigten Fettsäuren mit C₁₂ bis C₂₂, die mit linearen oder verzweigten primären Alkoholen mit C₃ bis C₁₂ verestert sind, ein oder mehrere Pflanzenöl(e), ein oder mehrere Silikonöl(e) und/oder deren Mischungen ist/sind, wobei das eine oder die mehreren Öl(e), das/die bei 25 °C und Atmosphärendruck flüssig ist/sind, bevorzugt Mineralöl, flüssiges Paraffin (Paraffinum perliquidum), pastöses Paraffin (Paraffinum subliquidum), Polydecen, Octylpalmitat, Isocetylpalmitat, Isopropylpalmitat, Octylstearat, Oleyloleat, Myristylmyristat, Mandelöl, Avocadoöl, Kokosnussöl, Maisöl, Baumwollsamenöl, Leinsamenöl, Traubenkernöl, Hanföl, Olivenöl, Palmkernöl, Erdnussöl, Kürbiskernöl, Reiskleieöl, Saflorsamenöl, Sesamsamenöl, Sonnenblumenkernöl, Sojabohnenöl, Walnussöl und/oder deren Mischungen ist/sind .

11. Zusammensetzung gemäß einem der Ansprüche 1 bis 8 und/oder 10, **dadurch gekennzeichnet, dass** die Zusammensetzung A eine Bleichpastenzusammensetzung ist.

12. Zusammensetzung gemäß einem der Ansprüche 1 bis 7 und/oder 10, **dadurch gekennzeichnet, dass** die Zusammensetzung A eine wässrige Zusammensetzung ist, die Wasser in einer Gesamtkonzentration von mehr als 10 Gew.-%, bevorzugt 30 Gew.-% oder mehr, bevorzugter 50 Gew.-% oder mehr, bezogen auf das Gesamtgewicht der Zusammensetzung A, umfasst, und der pH-Wert der Zusammensetzung A bevorzugt im Bereich von 7 bis 12, bevorzugter im Bereich von 8 bis 11 und noch bevorzugter im Bereich von 8,5 bis 10,5 liegt.

13. Zusammensetzung gemäß einem der vorhergehenden Ansprüche, **dadurch gekennzeichnet, dass** die Zusammensetzung A ein oder mehrere wasserlösliche(s) Verdickungspolymer(e) umfasst, bevorzugt ein oder mehrere wasserlösliche(s) anionische(s) oder nichtionische(s) Verdickungspolymer(e), bevorzugter ein oder mehrere Polymer(e), das/die Einheiten aus Acrylat, Methacrylat, Methacrylamid umfasst/umfassen, und/oder ein oder mehrere Xanthangummi, ein oder mehrere Dehydroxanthangummi und/oder ein oder mehrere Cellulosepolymer(e) wie Methylcellulose, Ethylcellulose, Hydroxyethylcellulose, Hydroxypropylmethylcellulose, ein oder mehrere Alginatpolymer(e) und/oder deren Salz(e) und/oder deren Mischungen.

14. Bausatz zum Bleichen und/oder Aufhellen von Keratinfasern, bevorzugt menschlichen Keratinfasern, bevorzugter menschlichem Haar, umfassend:
- eine Zusammensetzung A, wie in einem der Ansprüche 1 bis 13 definiert, und
- eine Zusammensetzung B, umfassend ein oder mehrere Oxidationsmittel, bevorzugt Wasserstoffperoxid.

15. Verfahren zum Bleichen und/oder Aufhellen von Keratinfasern, bevorzugt menschlichen Keratinfasern, bevorzugter menschlichem Haar, umfassend die folgenden Schritte:
i) Mischen einer Zusammensetzung A, wie in einem der Ansprüche 1 bis 13 definiert, mit einer Zusammensetzung B, wie in Anspruch 14 definiert, um eine gebrauchsfertige Zusammensetzung mit einem pH-Wert im Bereich von 7 bis 12 zu erhalten,
ii) Auftragen der gebrauchsfertigen Zusammensetzung auf Keratinfasern und Einwirkenlassen,
iii) Ausspülen der gebrauchsfertigen Zusammensetzung aus den Keratinfasern.

## Revendications

1. Composition A de blanchiment et/ou d'éclaircissement pour fibres kératiniques, de préférence fibres kératiniques humaines, plus préférentiellement cheveux humains, comprenant :
a) du tris-(hydroxyméthyl)-aminométhane et/ou du 2-amino-2-méthyl-1,3-propanediol, et/ou leur(s) sel(s), et/ou leurs mélanges,
b) un ou plusieurs agents alcalinisants inorganiques,
c) un ou plusieurs sels d'ammonium ou de métal alcalin ou de métal alcalino-terreux de persulfates ou de peroxysulfates.

2. Composition selon la revendication 1 **caractérisée en ce que** le ou les composé(s) selon le groupe a) est/sont du tris-(hydroxyméthyl)-aminométhane, et/ou ses sel(s).

3. Composition selon l'une quelconque des revendications 1 et/ou 2 **caractérisée en ce que** la concentration totale du/des composé(s) selon le groupe a) est dans la plage de 0,5 % à 20 % en poids, de préférence dans la plage de 0,75 % à 15 % en poids, plus préférablement dans la plage de 1 % à 12,5 % en poids, calculée par rapport au poids total de la composition A.

4. Composition selon l'une quelconque des revendications précédentes **caractérisée en ce que** le ou les agent(s) alcalinisant(s) inorganique(s) selon le groupe b) est/sont un sel de métal alcalin ou de métal alcalino-terreux d'un ou plusieurs métasilicates, d'un ou plusieurs silicates, d'un ou plusieurs disilicates, d'un ou plusieurs hydroxydes, d'un ou plusieurs phosphates, d'un ou plusieurs carbonates, d'un ou plusieurs bicarbonates, et/ou leurs mélanges, de préférence le ou les agent(s) alcalinisant(s) inorganique(s) selon le groupe b) est/sont un sel de métal alcalin ou de métal alcalino-terreux d'un ou plusieurs métasilicates, d'un ou plusieurs carbonates, d'un ou plusieurs bicarbonates, et/ou leurs mélanges, plus préférablement un ou plusieurs agent(s) alcalinisant(s) inorganique(s) selon le groupe b) est/sont du métasilicate de sodium, du carbonate de sodium, du bicarbonate de sodium, et/ou leurs mélanges.

5. Composition selon l'une quelconque des revendications précédentes **caractérisée en ce que** la concentration totale du/des composé(s) selon le groupe b) dans la composition A est dans la plage de 0,25 % à 30 % en poids, de préférence dans la plage de 0,5 % à 25 % en poids, plus préférablement dans la plage de 1 % à 20 % en poids, de manière davantage préférée dans la plage de 3 % à 18 % en poids, calculée par rapport au poids total de la composition A.

6. Composition selon l'une quelconque des revendications précédentes **caractérisée en ce que** le ou les composé(s) selon le groupe c) est/sont le persulfate de sodium, le persulfate de potassium, le persulfate d'ammonium, le peroxymonosulfate de sodium, le peroxymonosulfate de potassium, et le peroxymonosulfate d'ammonium, et/ou leurs mélanges, et de préférence la concentration totale du/des composé(s) selon le groupe c) dans la composition A est dans la plage de 1 % à 80 % en poids, de préférence dans la plage de 2 % à 70 % en poids, plus préférablement dans la plage de 5 % à 60 % en poids, calculée par rapport au poids total de la composition A.

7. Composition selon l'une quelconque des revendications précédentes **caractérisée en ce que** le rapport en poids du/des composé(s) selon le groupe a) au/aux composé(s) selon le groupe b) est dans la plage de 0,1 à 10, de préférence dans la plage de 0,2 à 8, plus préférentiellement dans la plage de 0,25 à 6.

8. Composition selon l'une quelconque des revendications précédentes **caractérisée en ce que** la concentration totale d'eau dans la composition A est de 10 % en poids ou moins, de préférence de 5 % en poids ou moins, plus préférablement de 1 % en poids ou moins, de manière davantage préférée de 0,5 % en poids ou moins, calculée par rapport au poids total de la composition A, de manière davantage préférée la composition A est une composition anhydre.

9. Composition selon l'une quelconque des revendications précédentes **caractérisée en ce que** la composition A est une poudre de blanchiment, et comprend de préférence un ou plusieurs excipient(s) pulvérulent(s), plus préférentiellement un ou plusieurs excipient(s) organique(s) ou inorganique(s), de manière davantage préférée un ou plusieurs de la terre de diatomées, du kaolin, de la bentonite, de l'amidon en particulier de maïs, de tapioca, de riz, de blé et de pomme de terre, de la poudre de nylon, de la montmorillonite, du gypse, de la sciure de bois et de la perlite, et/ou leurs mélanges.

10. Composition selon l'une quelconque des revendications précédentes **caractérisée en ce que** la composition A comprend une ou plusieurs huile(s) étant liquide(s) à 25 °C et à la pression atmosphérique, de préférence la ou les huile(s) est/sont une ou plusieurs huile(s) hydrocarbonée(s), une ou plusieurs huile(s) ester(s) telles que des esters d'acides gras constitués d'acides gras linéaires ou ramifiés, saturés ou insaturés, en C₁₂ à C₂₂ estérifiés avec des alcools primaires linéaires ou ramifiés en C₃ à C₁₂, une ou plusieurs huile(s) végétale(s), une ou plusieurs huile(s) de silicone, et/ou leurs mélanges, de préférence la ou les huile(s) étant liquide(s) à 25 °C et à la pression atmosphérique est/sont l'huile minérale, le paraffinum perliquidum, le paraffinum subliquidum, les polydécènes, le palmitate d'octyle, le palmitate d'isocétyle, le palmitate d'isopropyle, le stéarate d'octyle, l'oléate d'oléyle, le myristate de myristyle, l'huile d'amande, l'huile d'avocat, l'huile de noix de coco, l'huile de maïs, l'huile de graines de coton, l'huile de graines de lin, l'huile de pépins de raisin, l'huile de chanvre, l'huile d'olive, l'huile de palmiste, l'huile d'arachide, l'huile de graines de courge, l'huile de son de riz, l'huile de graines de carthame, l'huile de sésame, l'huile de graines de tournesol, l'huile de soja, l'huile de noix, et/ou leurs mélanges.

11. Composition selon l'une quelconque des revendications 1 à 8 et/ou 10 **caractérisée en ce que** la composition A est une pâte de blanchiment.

12. Composition selon l'une quelconque des revendications 1 à 7 et/ou 10 **caractérisée en ce que** la composition A est une composition aqueuse comprenant de l'eau à une concentration totale de plus de 10 % en poids, de préférence à 30 % en poids ou plus, plus préférablement à 50 % en poids ou plus, calculée par rapport au poids total de la composition A, et de préférence le pH de la composition A est dans la plage de 7 à 12, plus préférentiellement dans la plage de 8 à 11, encore plus préférentiellement dans la plage de 8,5 à 10,5.

13. Composition selon l'une quelconque des revendications précédentes **caractérisée en ce que** la composition A comprend un ou plusieurs polymère(s) épaississant(s) hydrosoluble(s), de préférence un ou plusieurs polymère(s) épaississant(s) hydrosoluble(s) anionique(s) ou non ionique(s), encore plus préférentiellement un ou plusieurs polymères comprenant des unités d'acrylate, de méthacrylate, de méthacrylamide, et/ou une ou plusieurs de gomme xanthane, un ou plusieurs de gomme déshydroxanthane, et/ou un ou plusieurs polymère(s) cellulosique(s) tels que la méthylcellulose, l'éthylcellulose, l'hydroxyéthylcellulose, l'hydroxypropylméthylcellulose, un ou plusieurs polymères d'alginate, et/ou leurs sels, et/ou leurs mélanges.

14. Kit à plusieurs composants pour le blanchiment et/ou l'éclaircissement de fibres kératiniques, de préférence fibres kératiniques humaines, plus préférentiellement cheveux humains, comprenant :
- une composition A telle que définie dans l'une quelconque des revendications 1 à 13, et
- une composition B comprenant un ou plusieurs agent(s) oxydant(s), de préférence du peroxyde d'hydrogène.

15. Procédé de blanchiment et/ou d'éclaircissement de fibres kératiniques, de préférence fibres kératiniques humaines, plus préférentiellement cheveux humains, comprenant les étapes consistant à :
i) mélanger une composition A telle que définie dans l'une quelconque des revendications 1 à 13 avec une composition B telle que définie dans la revendication 14 pour donner une composition prête à l'emploi présentant un pH dans la plage de 7 à 12,
ii) appliquer la composition prête à l'emploi sur des fibres kératiniques et la laisser,
iii) rincer la composition prête à l'emploi des fibres kératiniques.
